# EUROPEAN PATENT SPECIFICATION

(11) **EP 2 515 800 B1**
(45) Date of publication and mention of the grant of the patent: **29.06.2016**
(21) Application number: 10843422.6
(22) Date of filing: 23.11.2010
(51) Int. Cl.: A61F 2/24, B21D 11/20, B21D 26/06, B24C 1/10, B23K 26/00, C21D 7/06, C21D 10/00

(54) **METHOD OF PEENING METAL HEART VALVE STENTS**
VERFAHREN ZUM HÄMMERN VON METALL-HERZKLAPPENSTENTS
PROCÉDÉ DE MARTELAGE DE STENTS DE VALVULE CARDIAQUE EN MÉTAL

(30) Priority: 22.12.2009 US 289248 P; 19.11.2010 US 950799
(43) Date of publication of application: 31.10.2012
(73) Proprietor: Edwards Lifesciences Corporation, Irvine, CA 92614 (US)
(72) Inventor: DAVIDSON, James, A., Irvine, CA 92614 (US); CAMPBELL, Louis, A., Irvine, CA 92614 (US)
(74) Representative: Eisenführ Speiser
(86) International application number: PCT/US2010/057788
(87) International publication number: WO 2011/087582

(56) References cited:
- EP-A1- 0 985 384
- EP-A1- 0 985 384
- WO-A1-2009/106545
- WO-A2-2008/048679
- US-A1- 2005 182 478
- US-A1- 2005 182 478
- US-A1- 2008 154 355
- US-A1- 2009 216 312

## Description

### FIELD

The present disclosure relates to medical devices and methods for manufacturing medical devices. Specifically, the disclosure relates to improved heart valve stents and methods of manufacturing the same.

### BACKGROUND

In minimally invasive surgery techniques, medical devices, such as prosthetic heart valves, can be crimped or otherwise radially compressed onto a delivery catheter, positioned within a patient's heart, and then expanded. Medical devices that are implanted in this manner are typically guided through small and circuitous vessels in order to reach their destinations. During delivery, the devices typically must be flexible enough to undergo some bending while maneuvering through the vessel, and yet resist the formation of cracks. Smaller profiles while in the compressed state allow for easier maneuvering through the patient's vessels. Thus, it is desirable for devices such as heart valve stents to be as small as possible, but without sacrificing mechanical characteristics and strength.

US2005182478 discloses a method of making Nitinol stents, guidewires, catheters, intravascular filters comprising shock peening at least a portion of an outer surface of the frame.

Stents and other medical devices made of biocompatible metal alloys, especially Nitinol, are commonly subject to fractures. Fractures, such as fatigue-related fractures, are problematic for the device's performance within the body. Further, scratches and other surface abnormalities and irregularities can lead to fatigue crack growth.

Some attempts have been made at combating these fracture problems in medical devices. For example, increasing the stiffness of the device can reduce its susceptibility to fractures, but, in the case of heart valve stents, can also undesirably increase shock loading to the valve leaflets. Other techniques such as annealing or work hardening the devices can reduce residual tensile stresses, but do not impact surface defects that can create crack initiation points.

Thus, an improved heart valve stent and method of forming heart valve stents are needed to increase the factor of safety (resistance to fatigue) without simultaneously increasing the stiffness and/or the thickness of the stent.

### SUMMARY

Disclosed methods can improve the fatigue safety factor, resistance to fatigue cracks, and/or the surface appearance of medical devices such as heart valve stents, without increasing the stiffness or thickness of the stent. According to some disclosed embodiments, thinner stents having substantially the same fatigue life as thicker stents can be formed, thus allowing for lower profiles when the heart valve stent is radially compressed or crimped onto a delivery system, and requiring less material for manufacturing the stent.

One method of improving fatigue resistance in a medical device comprises providing a frame (*e.g*., an annular frame such as an annular heart valve frame), wherein the frame comprises an inner and an outer surface, peening at least a portion of both inner and outer surfaces of the frame, thereby improving fatigue resistance of the frame, and securing the frame to one or more leaflets, such as to form a prosthetic heart valve. Such methods can be particularly useful for, for example, percutaneous heart valve stents comprising shape memory materials, such as Nitinol.

Peening can comprise shot peening, laser peening, and/or ultrasonic peening. Shot peening can be done with biocompatible shots in some embodiments. Similarly, laser peening can comprise using a sacrificial overlay layer over at least a portion of the frame, wherein the overlay layer is arranged between the frame and a laser peening source, and the overlay layer does not compromise biocompatibility of the device being peened.

In some methods, substantially the entire outer surface of the frame can be subject to one or more types of peening. Further, peening can be directed to one or more specific regions of the outer and inner surfaces of the frame. For example, peening can be directed to one or more areas of the inner and outer surfaces of the frame that are subject to the greatest stresses or risk of fatigue crack growth.

Disclosed methods can also alter surface characteristics of medical devices, such as by improving the device's aesthetic appearance and/or substantially eliminating or reducing score lines or burrs. One method of altering surface characteristics of a medical device comprises providing a radially collapsible and expandable frame, wherein the frame comprises an inner and an outer surface, and peening at least a portion of both inner and outer surfaces of the frame, thereby altering one or more surface characteristics of the frame, and securing the frame to one or more valve leaflets. For example, peening can uniformly increase surface roughness, thereby masking or removing surface irregularities such as score lines, scratches, notches, and/or burrs. In one method, peening comprises roughening at least a portion of both inner and outer surfaces of the frame with uniformity sufficient to mask one or more surface scratches, notches, and/or burrs. The surface characteristics of at least a portion of the inner surface of the frame can be altered by peening, in addition to at least a portion of the outer surface of the frame.

Peening can comprise one or more of shot peening with biocompatible beads, laser peening with a biocompatible overlay layer, and ultrasonic peening.

Methods of peening medical devices are also disclosed. One method for peening a medical device comprises providing a radially collapsible and expandable frame, wherein the frame comprises an inner and an outer surface, placing the frame on a stabilizing device, peening at least a portion of both inner and outer surfaces of the frame, thereby altering one or more surface characteristics of the frame, and coupling the frame to a prosthetic heart valve for implantation in a human subject. Placing the frame on a stabilizing device, such as securing or mounting the frame on a mandrel or a delivery system component (*e.g*., a guidewire or delivery catheter) can aid in positioning the frame appropriately for peening. For example, a frame can be held substantially stationary on a mandrel, while a shot peening nozzle moves around and over the surface of the frame. Similarly, a frame can be secured in place on a mandrel while a laser beam is directed over the surface of the frame for laser peening.

The frame can be coupled to the rest of the prosthetic heart valve (*e.g*., a leaflet structure) after one or more peening processes have been performed. In one embodiment, the frame can be coupled to a leaflet structure and a skirt to form a complete prosthetic heart valve, the valve can be crimped onto a delivery system component such as a guidewire (*e.g*., the frame is coupled to the prosthetic valve prior to being placed on the delivery system component), and the frame can be peened with one or more peening techniques before delivering the prosthetic valve to the patient. The frame can be subjected to one or more peening processes before the prosthetic valve is crimped onto the delivery system component and/or before being coupled to other prosthetic heart valve components. For example, a self-expandable Nitinol frame can be subjected to one or more peening processes, placed onto a delivery system component, and then radially compressed, such as with a restraining sheath, for implantation into a patient.

The foregoing and other objects, features, and advantages of the invention will become more apparent from the following detailed description, which proceeds with reference to the accompanying figures.

### BRIEF DESCRIPTION OF THE DRAWINGS

FIG. 1 illustrates an exemplary prosthetic heart valve including a stent.
FIG. 2 illustrates a heart valve stent.
FIG. 3 illustrates another embodiment of a prosthetic heart valve including a stent.
FIG. 4 is a flow chart of one method of peening a metal heart valve stent.

### DETAILED DESCRIPTION

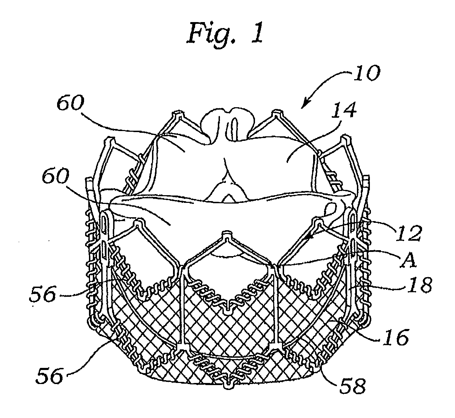

FIG. 1 illustrates an exemplary prosthetic heart valve 10 having a stent, or frame 12. Such exemplary valve is the subject of co-pending application number 12/480,603, which is hereby incorporated herein in its entirety. Valve 10 and frame 12 are configured to be radially collapsible to a collapsed or crimped state for introduction into the body on a delivery catheter and radially expandable to an expanded state for implanting the valve at a desired location in the body (*e.g*., the native aortic valve). Frame 12 can be made of a plastically-expandable material that permits crimping of the valve to a smaller profile for delivery and expansion of the valve using an expansion device such as the balloon of a balloon catheter. Alternatively, valve 10 can be a so-called self-expanding valve wherein the frame 12 is made of a self-expanding shape memory material such as Nitinol. A self-expanding valve can be crimped to a smaller profile and held in the crimped state with a restraining device such as a sheath covering the valve. When the valve is positioned at or near the target site, the restraining device is removed to allow the valve to self-expand to its expanded, functional size.

Implantable prosthetic valve 10 comprises a radially collapsible and expandable frame or stent 12 and a leaflet structure 14 comprising a plurality of leaflets 60. The leaflet structure has a scalloped lower edge portion that is positioned inside of and secured to the frame 12. The valve can further include an annular skirt member 16, which can be disposed between the frame 12 and the leaflet structure 14 such that the scalloped lower edge portion can be attached to an inner surface of the skirt member 16. Skirt 16 can be secured to the inside of frame 12 via sutures 56. Suture 58 can secure the leaflet structure 14 to skirt 16. Each leaflet can have an upper edge, a curved lower edge and two side flaps extending between respective ends of the upper edge and the lower edge, wherein each side flap is secured to an adjacent side flap of another leaflet to form commissures of the leaflet structure. Each commissure can be attached to one of the commissure attachment posts, and a reinforcing bar can be positioned against each side flap for reinforcing the attachments between the commissures and the commissure attachment posts.

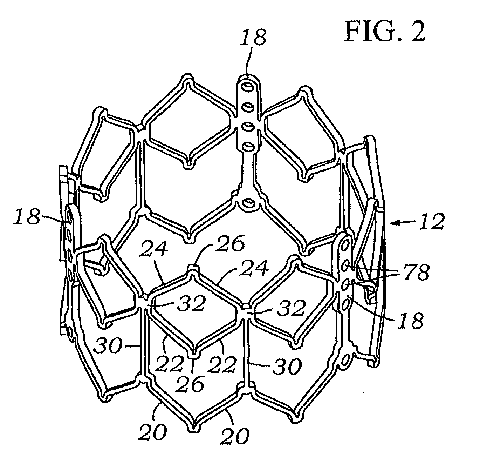

An exemplary frame 12, which is shown alone in FIG. 2 for illustrative purposes, can comprise a plurality of angularly spaced, axial struts or posts 18 that are interconnected by a plurality of rows of circumferential struts. Posts 18 can be interconnected via a lower row of circumferentially extending struts 20 and first and second rows upper rows of circumferentially extending struts 22 and 24, respectively. The struts in each row desirably are arranged in a zigzag or generally saw-tooth like pattern extending in the direction of the circumference of the frame as shown. Adjacent struts in the same row can be interconnected to one another as shown in FIGS. 1 and 2 to form an angle A. Each pair of struts 22 connected at a common crown structure 26 forms a cell with an adjacent pair of struts 24 in the row above. Each cell can be connected to an adjacent cell at a node 32. Each node 32 can be interconnected with the lower row of struts by a respective vertical (axial) strut 30 that is connected to and extends between a respective node 32 and a location on the lower row of struts 20 where two struts are connected at their ends opposite crown structures 26. Openings 78 in posts 18 can be used, among other things, to secure frame 12 to the leaflet structure.

The prosthetic heart valve shown in FIGS. 1 and 2 is meant to be one example, and not limiting in any way. As used throughout this disclosure, the term "frame" can be any type of frame suitable for use with a medical device such as a heart valve. Frames can be substantially annular in some embodiments. In some embodiments, a frame can be shaped from one or more arcs that are joined together.

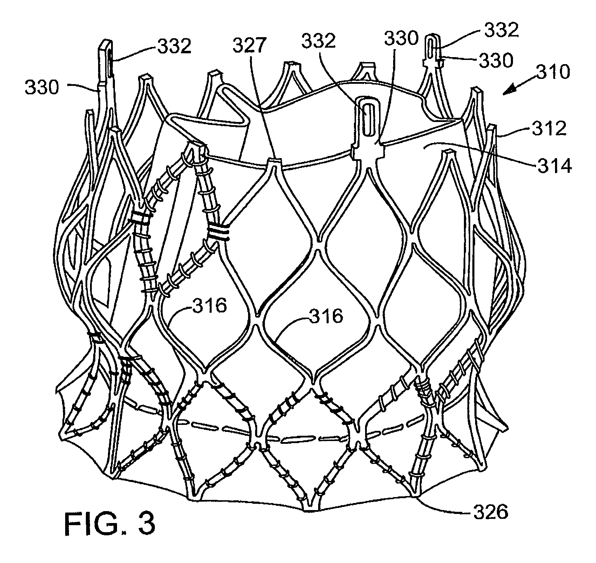

Another embodiment of an exemplary medical device is the self-expandable prosthetic heart valve shown in FIG. 3. Such exemplary valve is the subject of co-pending application number 12/429,040, which is hereby incorporated herein in its entirety. The valve 310 includes an expandable frame member, or stent, 312 that supports a flexible leaflet section 314. The valve 310 is radially compressible to a compressed state for delivery through the body to a deployment site and expandable to its functional size shown in FIG. 3 at the deployment site. In certain embodiments, the valve 310 is self-expanding; that is, the valve can radially expand to its functional size when advanced from the distal end of a delivery sheath.

As shown, the stent 312 can be formed from a plurality of longitudinally extending, generally sinusoidal shaped frame members, or struts, 316. The struts 316 are formed with alternating bends and are welded or otherwise secured to each other at nodes formed from the vertices of adjacent bends so as to form a mesh structure. The struts 316 can be made of a suitable shape memory material, such as the nickel titanium alloy known as Nitinol, that allows the valve to be compressed to a reduced diameter for delivery in a delivery apparatus and then causes the valve to expand to its functional size inside the patient's body when deployed from the delivery apparatus.

The stent 312 of FIG. 3 has an inflow end 326 and an outflow end 327. The stent 312 can have a plurality of angularly spaced retaining arms, or projections, in the form of posts 330 (three in the illustrated embodiment) that extend from the stent upper portion, near the outflow end 327. Each retaining arm 330 has a respective aperture 332 that is sized to receive prongs of a valve-retaining mechanism that can be used to form a releasable connection between the valve and a delivery apparatus. In alternative embodiments, the retaining arms 330 need not be provided if a valve-retaining mechanism is not used.

Medical devices, such as the percutaneous heart valve stents shown in FIGS. 1-3, can comprise any suitable material, and preferably comprise a biocompatible metal alloy. Suitable materials can include Nitinol, other nickel-titanium alloys, copper, aluminum, titanium, nickel, platinum, tantalum, cobalt, chromium, cobalt-chromium alloys, steel-based alloys such as stainless steel, nickel-based alloys (*e.g*., a nickel-cobalt-chromium alloy such as MP35N™), polymers, or combinations or alloys thereof.

Disclosed improved heart valve stents and methods for making the same can allow the manufacture of thinner heart valve stents, using less material to achieve the same or better performance in radial and crush force resistance, fatigue resistance (*e.g*., fatigue safety factor), and/or corrosion resistance. Moreover, the crimped profile of the frame can be reduced, thereby providing a lower profile valve assembly for percutaneous delivery to the treatment location in the body. While the methods are disclosed with reference to medical devices that are collapsible and expandable, the methods are equally applicable to non-collapsible medical devices, such as the stents of non-collapsible surgical valves. For example, the methods disclosed herein can be used to treat the metal stent or wire form of the valve disclosed in U.S. Patent No. 6,585,766, which is incorporated herein by reference.

One such method comprises peening to modify the mechanical properties of heart valve stents. Peening can produce a compressive residual stress layer within the stent and/or on the surface of the stent that can increase fatigue life. Generally, the surface of a heart valve stent can be shot peened by being mechanically impacted or bombarded with a plurality of shots, such as small metal, glass, aluminum oxide, or ceramic beads, spheres, or pellets, walnut shells, or similar materials. In some embodiments, the surface of a heart valve stent can be subject to sand and/or grit blasting. In embodiments of peening a heart valve stent, the material (*e.g*., the shots) can be forced, such as forced by compressed air through a nozzle, onto the surface of a metal stent so as to impact the surface and create plastic deformation in at least some regions of the stent. Such impact on the surface can alter the structure of the stent near the surface, such as, for example, by creating compressive strains in the peened surface. Shot peening can result in favorable compressive residual stress or reduce pre-existing residual tensile stress, thus improving fatigue strength or fatigue life.

For example, in some embodiments, the determined fatigue safety factor of a heart valve stent can be increased from about 1.0 to a fatigue safety factor greater than about 1.0. In some embodiments, the fatigue safety factor can be increased from about 1.0 prior to peening, to about 1.05 or greater, to about 1.2 or greater, or to about 1.4 or greater.

At least some portion of the stent can be peened in the disclosed embodiments, where the terms "peening" and "peened" include shot peening, laser peening, and ultrasonic peening. In some embodiments, substantially the entire surface of the heart valve stent can be subject to peening. In other embodiments, only certain regions of the stent are subject to peening. For example, in some embodiments, peening can be focused on the crown structures 26 and the nodes 32 of FIGS. 1-2. In some embodiments, peening can be focused on the axial posts 18 of FIGS. 1-2. In some embodiments, regions of the heart valve stent that are most susceptible to fatigue cracks can be peened to a greater extent than other regions of the heart valve stent. In some embodiments, a peening mask can be used to prevent specific regions of the heart valve stent from being impacted by the shots and/or laser.

Disclosed methods of shot peening heart valve stents can include varying the size, shape, hardness, and/or material of the shots, the speed of the shots, the nozzle pressure firing the pellets, the nozzle distance from the sample being peened, and/or the duration of the process. Varying these parameters can allow at least some control over the results of the peening. For example, increasing the mass and/or incident velocity of the pellets can increase the magnitude of the compressive stress imparted to the stent material. Higher pellet mass and/or velocity can also allow the process to obscure more significant surface defects that otherwise can reduce the fatigue resistance of the stent. In some embodiments, increasing the pellet mass and/or velocity can be limited so as to substantially prevent dimensional distortion of the stent such as warping the stent or removing too much material. The appropriate particle hardness, mass, and velocity can be determined through an empirical process for any given stent material and design configuration.

The working distance (*e.g*., the nozzle distance from the sample being peened) can also be varied. Reducing the working distance can increase the effects of the peening. Changing one or more parameters at a time can have various impacts on the effects of the peening. For example using a shot or pellet comprising a material of relatively low hardness can reduce the effects of the peening as compared to using a shot or pellet comprising a material of relatively high hardness. However, the effectiveness of peening with a lower hardness pellet can be increased by, for example, varying the velocity (*e.g*., increasing the velocity) of the pellet and/or varying the working distance (*e.g*., decreasing the working distance). Likewise, in some embodiments, the velocity of the pellets can be decreased and/or the working distance can be increased in order to lessen the effects of the peening.

In some embodiments, process parameters can be varied to account for system requirements or limitations. For example, if a peening system can only accommodate a relatively short working distance, varying other parameters can change the effects of the peening to overcome this limitation. For example, if the working distance is not as great as desired, parameters such as the pellet velocity can be increased to increase the effects of the peening. Each of the process parameters can be increased or decreased in order to create the desired effects on the piece being peened.

In some embodiments, the parameters of the process can be varied for different portions of the stent. For example, some regions of the stent can be subject to peening with smaller beads at lower pressure and/or for less time than other regions of the stent. The shot size, velocity, and/or peening duration can be varied to result in desirable combinations of residual surface stress and surface appearance. In some embodiments, areas of the heart valve stent subject to tensile stress while the valve is in use can be selectively peened, while areas of the heart valve stent not subject to surface tensile stress while in use can have either reduced or no peening. Such selective peening can be useful in obtaining sufficient surface modification to improve fatigue resistance while minimizing dimensional distortion of the stent.

For example, shot diameter can be varied from less than about 10 microns to greater than about 50 microns. Nozzle pressure can be varied from less than about 10 psi to greater than about 100 psi, with specific embodiments using nozzle pressure between about 30 psi and about 80 psi. One or more nozzles can be used in some methods. For example, in one method, a three nozzle array can be used to shot peen a heart valve stent. The one or more nozzles can be held at a constant distance from the stent, or the distance can be varied throughout the duration of the peening. In some embodiments, the nozzle or nozzles can be from about 0.5 cm or less to about 10 cm or greater away from the stent. In some embodiments, peening can be performed for less than about 1 second to greater than about 5 seconds. Peening can be performed for a period of time long enough to achieve desired result. For example, it can be performed for a period of time long enough to achieve a desired surface roughness, such as a roughness of from about 50 nm to about 500 nm. In some embodiments, the heart valve stent can be displaced and/or rotated from its original position when peening was started. For example, the heart valve stent can be rotated a certain number of degrees at a time, for example, 30, 60, 90, or 120 degrees at a time, and peening repeated after each rotation until the stent has been rotated substantially 360 degrees and peened on all desired surfaces.

The heart valve stent can be mounted onto a stabilizing structure such as a mandrel to protect the stent's inner surface during peening. The stent can be rotated while the shots are projected towards the outer surface of the stent from a pressurized shot source. Similarly, the stent can be rotated while one or more laser beams are projected towards the outer surface of the stent from a laser peening apparatus. In some embodiments, one or more nozzles and/or laser beams can be moved across and/or around the stent. The one or more nozzles and/or laser beams can be positioned at an angle to the vertical axis of the stent, such as at an angle of approximately 30 degrees to the vertical axis of stent. After being peened, the stent can be removed from the mandrel and cleaned, before being secured to one or more valve leaflets. In alternative embodiments, the stent can be secured in place such that at least a portion of both the inner and outer surfaces of the stent are subject to peening. For example, a heart valve stent can be secured by one or more of the posts 18 and at least some portion of the inner and/or outer surfaces subjected to peening.

In some disclosed methods, a heart valve stent can be peened before attachment to other components of a prosthetic valve (*e.g*., the leaflet structure 14 and the skirt 16). In alternative embodiments, the stent can be peened after attachment to one or more of the other components of a prosthetic valve. Some methods provide for peening the heart valve stent before crimping it onto a delivery system.

In some embodiments, a heart valve stent can be annealed and/or subject to some other hardening technique before peening. Such annealing can leave residual stresses on the stent surface after the stent is formed. Peening can create compressive surface stress which can be beneficial to the fatigue life of the heart valve stent, and help to overcome the disadvantageous stresses resulting from processes such as annealing. Thus, some methods of peening heart valve stents can reduce adverse residual stress on the surface of metallic stents as well as add favorable residual stresses to surface irregularities, rendering them less detrimental from a fatigue life perspective.

Cold drawing a wire used to form a stent structure can result in surface damage or irregularities such as score lines, scratches, notches, and/or intermittent burrs. Disclosed methods of peening a heart valve stent can also aesthetically improve the stent's surface appearance, such as by substantially eliminating or reducing such surface irregularities. While peening can result in what optically appears to be a rougher surface, peening can actually result in a smoother surface based on quantitative measurement methods such as profilometry.

Disclosed methods of peening heart valve stent surfaces can create a more uniform surface that can improve fatigue life. In some embodiments, peening the surface of a heart valve stent can alter the surface of the stent, such as by removing stress risers. In some embodiments, uniformly peening the surface can mask the surface irregularities and result in improved fatigue resistance. In some embodiments, application of the shots and/or laser beams is substantially uniform over the entire surface of the heart valve stent.

Furthermore, in some methods, peening can reduce deleterious effects of processing techniques such as plating, decarburization, corrosion, and grinding.

In these ways, the present disclosure can allow for the use of a thinner stent, using less material (*e.g*., a heart valve stent with a smaller profile in its crimped state) without sacrificing fatigue resistance. A lower profile stent can save money on materials required, and can also provide more flexibility and easier tracking through the patient's vasculature during delivery. As a result, complications associated with the incision site and implantation can be reduced. Thinner stents can also reduce the external diameter of a valve and/or allow increased flow area through the valve for any given external diameter. Increased flow area can reduce the work required by the patient's heart to pump blood through the valve, thereby contributing to heart valve optimization.

In some embodiments, peening does not affect the sterility and/or biocompatibility of the stent surface. In these embodiments, peening can be performed such that surface contamination is substantially avoided. In some embodiments, peening can be used to remove at least some adherent surface contaminants. Further, in some methods, the shots can comprise biocompatible beads, such as biocompatible metal or ceramic beads. Different bead materials can be used depending on the material of the heart valve stent, if desired. For example, a particular heart valve stent can be shot peened using shots selected to be substantially the same or a similar material as the heart valve stent itself. Similarly, a particular heart valve stent can be shot peened using shots selected to possess different mechanical characteristics from the heart valve stent material. For example, selected shots can have a greater or lesser hardness than the heart valve stent material, to create desired results.

Some disclosed methods comprise using peening techniques such as laser peening and/or ultrasonic peening. Laser peening can create a highpressure plasma that generates a shock wave that can force the resulting compressive stress deeper into the surface of the stent. In some embodiments, a tamping layer, such as a laminar flow of water, can be provided over the surface of the stent during peening. The water layer can substantially prevent the plasma formed by laser peening from expanding, and thus can help drive the energy into the stent surface.

Embodiments of laser peening a medical device can include directing a laser beam onto a sacrificial overlay layer to induce a pressure shock wave within the device that can be used to produce one or more compressive residual stress regions on the surface of and/or within the heart valve stent. Some methods of laser peening a medical device such as a heart valve stent can comprise using a sacrificial overlay or ablative layer, such as paint, HDPE, tape, or any suitable biocompatible material. A laser apparatus that includes a laser source having a beam intensity sufficient to induce a pressure shock wave within the stent can be used to direct one or more laser beams onto the stent surface or onto the sacrificial overlay layer to produce one or more residual stress regions within the stent. The overlay layer can absorb radiation and act as a thermal barrier to protect the stent from thermal effects generated during the laser peening process. In preferred embodiments, the overlay layer comprises one or more biocompatible materials that substantially do not compromise the biocompatibility and/or sterility of the heart valve stent. In some embodiments, the overlay layer can be patterned, to provide for laser peening of selected regions of the heart valve stent, while preventing other surfaces from being peened, such as by preventing transmission of the shock wave into those portions of the stent.

Embodiments of ultrasonic peening a medical device can include using a mist of microbeads inside a vibrating chamber. Methods can combine high frequency impacts of these microbeads or strikers with ultrasonic oscillation of the medical device. Methods of ultrasonic peening can include bringing an acoustically tuned body to resonance by energizing an ultrasonic transducer. The energy generated from these high frequency impulses can be imparted to the surface of a medical device through the contact of specially designed steel pins. Transfer pins can be free to move axially between the resonant body and the surface of the medical device (*e.g*., the heart valve stent). Bringing the ultrasonic transducer, pins, and other components into contact with a medical device can acoustically couple them together, creating harmonic resonance that can result in compressive residual stress, stress relief, and surface improvements.

As with shot peening, parameters of laser peening and ultrasonic peening can be varied to achieve different desired results. For example, different wavelengths of lasers can be used for different peening processes, or the frequency and/or wavelength can be varied during a single peening process. In some embodiments, laser beams can be provided having a frequency of from about 3 Hz or less to about 6 Hz or greater. Frequencies for ultrasonic peening can range from about less than 25 kHz to greater than about 55 kHz. The displacement amplitude of the resonant body can also be varied, such as between about 20 microns and about 50 microns. The above parameters as well as other parameters known in the art can be varied and optimized for specific applications, such as will be appreciated by those skilled in the art.

In some methods, laser peening or ultrasonic peening alone can be used to improve fatigue resistance or surface properties of heart valve stents. In alternative methods, laser peening and ultrasonic peening can be used in combination with each other, and/or in combination with shot peening to produce desired results. Some methods can comprise cold straightening before, after, or during a peening technique. Robotics and/or CNC machines can aid in the peening process, whether shot peening, laser peening, and/or ultrasonic peening is used. For example, the nozzle used to force shots onto the surface of a heart valve stent can be robotically positioned and moved over the surface of the stent.

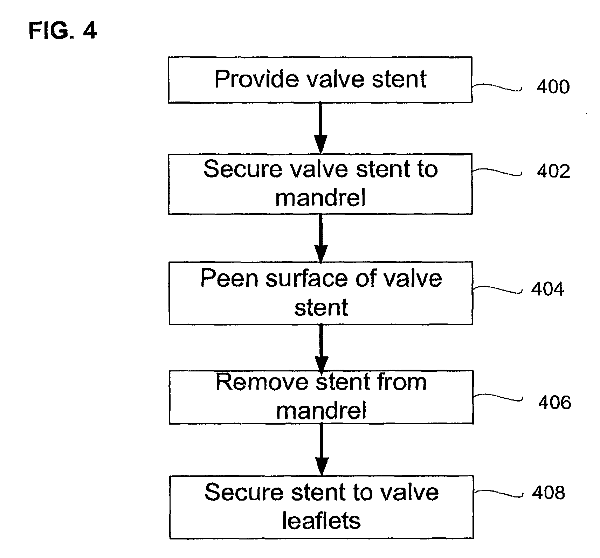

While, as described above, many different methods of peening a metal valve stent are disclosed, FIG. 4 illustrates a flow chart of one exemplary embodiment. A metal stent, such as a Nitinol heart valve stent can be provided (step 400). The valve stent can be secured to a mandrel, or otherwise held in place such that at least a portion of the inner and/or outer surface of the valve stent is accessible by a peening tool (step 402). At least a portion of the inner and outer surface of the valve stent can then be peened, such as by shot peening, laser peening, and/or ultrasonic peening (step 404). The valve stent can be rotated and/or repositioned to access different portions of the surface, if necessary. Once peening is complete, the valve stent can be removed from the mandrel (step 406). The valve stent can then be secured to a leaflet structure comprising one or more valve leaflets, without requiring any further processing (step 408). In this way, peening can be essentially the last processing step performed on the valve stent before securing the valve stent to one or more valve leaflets to form a prosthetic valve.

Independent from the detailed description of the several embodiments outlined above, it has to be noted that the following issues can advantageously be present on the several embodiment, alone or in combination with each other. In particular, the frame can be made of Nitinol. Alternatively or additionally, peening can comprise selectively peening only areas of the outer surface of the frame that are subject to tensile stress while in use. Further alternatively or additionally, peening can comprise one or more of shot peening with biocompatible beads, laser peening with a biocompatible overlay layer, and ultrasonic peening. Moreover, alternatively or additionally, peening can promise changing the roughness of at least a portion of the outer surface of the frame with uniformity sufficient to mask one or more surface scratches, notches, and/or burrs. Further, additionally or alternatively, that substantially only the portions of the frame that are subject to the greatest tensile stresses are penned.

## Claims

1. A method of making a prosthetic heart valve (10) comprising:
providing (400) a valve frame (12), wherein the frame (12) comprises an inner and an outer surface;
peening (404) at least a portion of the outer surface of the frame (12), thereby improving fatigue resistance of the frame (12); and
securing (408) valve leaflets (14, 60) to the frame (12);
wherein peening (404) comprises peening at least a portion of both the inner and outer surfaces of the frame (12).

2. The method according to claim 1, wherein peening (404) comprises shot peening with biocompatible beads.

3. The method according to claim 1, wherein peening (404) comprises changing the surface roughness of at least a portion of the outer surface of the frame (12) with uniformity to mask one or more surface scratches, notches, and/or burrs.

4. The method according to claim 1, wherein peening (404) comprises selectively peening areas of the surface of the frame (12) that are subject to tensile stress while in use.

5. The method according to claim 1, wherein peening (404) comprises at least one of laser peening and ultrasonic peening.

6. The method according to claim 1, wherein peening (404) comprises peening the entire outer surface of the frame (12).

7. The method according to claim 1, wherein peening (404) comprises altering one or more surface characteristics of the frame (12).

8. The method according to any of claims 1 to 7, wherein the frame (12) is a radially expandable and collapsible annular frame.

9. The method according to any of claims 1 to 8, wherein the frame (12) is made of a self-expanding, shape memory material; like Nitinol.

10. The method according claims 1 to 9, further comprising:
placing (402) the frame (12) on a stabilizing device.

## Patentansprüche

1. Verfahren zur Herstellung einer prothetischen Herzklappe (10), umfassend:
Vorsehen (400) eines Klappenrahmens (12), wobei der Rahmen (12) eine innere und eine äußere Oberfläche umfasst;
Hämmern (404) mindestens eines Teils der äußeren Oberfläche des Rahmens (12), wodurch die Ermüdungsbeständigkeit des Rahmens (12) verbessert wird; und
Befestigen (408) von Klappensegeln (14, 60) am Rahmen (12);
wobei Hämmern (404) mindestens Hämmern eines Teils sowohl der inneren als auch der äußeren Oberflächen des Rahmens (12) umfasst.

2. Verfahren nach Anspruch 1, wobei Hämmern (404) Strahlhämmern mit biokompatiblen Kugeln umfasst.

3. Verfahren nach Anspruch 1, wobei Hämmern (404) es umfasst, die Oberflächenrauheit mindestens eines Teils der äußeren Oberfläche des Rahmens (12) einheitlich zu ändern, um ein oder mehrere Kratzer, Nuten und/oder Grate in der Oberfläche zu beseitigen.

4. Verfahren nach Anspruch 1, wobei Hämmern (404) es umfasst, Bereiche der Oberfläche des Rahmens (12), die im Einsatz Zugbelastung ausgesetzt sind, selektiv zu hämmern.

5. Verfahren nach Anspruch 1, wobei Hämmern (404) mindestens eines von Laser-Peening und Ultraschall-Peening umfasst.

6. Verfahren nach Anspruch 1, wobei Hämmern (404) das Hämmern der gesamten äußeren Oberfläche des Rahmens (12) umfasst.

7. Verfahren nach Anspruch 1, wobei Hämmern (404) das Ändern einer oder mehrerer Oberflächen-Eigenschaften des Rahmens (12) umfasst.

8. Verfahren nach einem der Ansprüche 1 bis 7, wobei der Rahmen (12) ein radial expandierbarer und zusammenschiebbarer ringförmiger Rahmen ist.

9. Verfahren nach einem der Ansprüche 1 bis 8, wobei der Rahmen (12) aus einem selbstexpandierenden Material mit Formgedächtnis, wie etwa Nitinol, hergestellt ist.

10. Verfahren nach Anspruch 1 bis 9, weiter umfassend:
Platzieren (402) des Rahmens (12) auf einer stabilisierenden Vorrichtung.

## Revendications

1. Procédé pour faire une prothèse de valvule (10) cardiaque, comprenant:
se procurer (400) un cadre (12) de valvule, le cadre (12) comprenant une surface intérieure et une surface extérieure ;
marteler (404) au moins une partie de la surface extérieure du cadre (12) en améliorant ainsi la résistance à la fatigue du cadre (12) et
fixer (408) des valves (14, 60) au cadre (12) ;
le martelage (404) comprenant le martelage d'au moins une partie des deux surfaces intérieure et extérieure du cadre (12).

2. Procédé suivant la revendication 1, dans lequel marteler (404) comprend grenailler par des perles biocompatibles.

3. Procédé suivant la revendication 1, dans lequel marteler (404) comprend changer la rugosité superficielle d'au moins une partie de la surface extérieure du cadre (12) avec uniformité pour masquer une ou plusieurs rayures de surface, entailles et/ou bavures.

4. Procédé suivant la revendication 1, dans lequel marteler (404) comprend marteler sélectivement des zones de la surface du cadre (12), qui sont sujettes à des contraintes de traction en utilisation.

5. Procédé suivant la revendication 1, dans lequel marteler (404) comprend au moins un martelage au laser et un martelage ultrasonore.

6. Procédé suivant la revendication 1, dans lequel marteler (404) comprend marteler toute la surface extérieure du cadre (12).

7. Procédé suivant la revendication 1, dans lequel marteler (404) comprend vieillir une ou plusieurs caractéristiques de surface du cadre (12).

8. Procédé suivant l'une quelconque des revendications 1 à 7, dans lequel le cadre (12) est un cadre annulaire pouvant s'expanser et s'affaisser radialement.

9. Procédé suivant l'une quelconque des revendications 1 à 8, dans lequel le cadre (12) est en une matière à auto-expansion et à mémoire de forme, comme du nitinol.

10. Procédé suivant l'une des revendications 1 à 9, comprenant, en outre :
mettre (402) le cadre (12) sur un dispositif de stabilisation.
